# FASCICULE DE BREVET EUROPEEN

(11) **EP 2 648 635 B1**
(45) Date de publication et mention de la délivrance du brevet: **18.01.2017**
(21) Numéro de dépôt: 11807713.0
(22) Date de dépôt: 06.12.2011
(51) Int. Cl.: A61B 17/88

(54) **DISPOSITIF DE MISE EN TENSION D'UNE BANDE SOUPLE**
VORRICHTUNG ZUM SPANNEN EINES FLEXIBLEN BANDES
DEVICE FOR TENSIONING A FLEXIBLE BAND

(30) Priorité: 08.12.2010 FR 1004786
(43) Date de publication de la demande: 16.10.2013
(73) Titulaire: Implanet, 33650 Martillac (FR)
(72) Inventeur: BACCELLI, Christian, F-33650 Saucats (FR); LE COUEDIC, Régis, F-33900 Bordeaux (FR)
(74) Mandataire: Benech, Frédéric
(86) Numéro de dépôt international: PCT/FR2011/000639
(87) Numéro de publication internationale: WO 2012/076771

(56) Documents cités:
- WO-A1-2007/034112
- US-A- 1 346 940

## Description

La présente invention concerne un dispositif de mise en tension d'une bande souple pour maintien d'un élément osseux sur un implant, comportant une tige ayant une première extrémité munie de moyens d'appui sur l'implant, une pièce mobile d'accrochage de la bande souple sur une portion du dispositif et des moyens de blocage réglable de la pièce mobile en translation par rapport à ladite portion du dispositif.

Elle trouve une application particulièrement importante bien que non exclusive dans le domaine du redressement de la colonne vertébrale d'un patient présentant une courbure anormale.

Dans ce cas, les vertèbres n'étant pas alignées correctement les unes par rapport aux autres par rapport à l'axe vertébral, elles présentent des inclinaisons entre elles.

Afin de redresser l'ensemble, il est connu de remettre à une distance sensiblement équivalente des bords latéraux des vertèbres de part et d'autre de la colonne vertébrale, par le biais de tiges reliant entre elles, soit des vis, que l'on insère dans les vertèbres elles-mêmes, soit des crochets que l'on introduit le long du canal rachidien.

De tels dispositifs présentent cependant des inconvénients car ils sont agressifs.

Pour palier à ces inconvénients, il a été proposé un lien flexible de fixation de la vertèbre sur la pièce de liaison elle-même fixé à la tige de redressement.

Des moyens de blocage du lien flexible par refermeture de la pièce de liaison sur la tige sont alors nécessaires.

Le problème que cherche à résoudre l'invention est ici celui de la mise en tension de ce lien flexible de fixation de la vertèbre sur la pièce de liaison.

En effet, il est important que l'utilisateur lorsqu'il va mettre en place cette liaison puisse resserrer progressivement cette dernière sans pour autant endommager l'élément osseux qui vient donc être comprimé par la bande souple.

Il existe actuellement (WO 2007/034112) des ancillaires de mise en tension munis d'une tige d'une pièce mobile en translation le long de la tige et d'un élément monté sur la pièce mobile tendant horizontalement à écarter l'extrémité de la tige par rapport à la pièce.

Un dispositif avec les caractéristiques du préambule de la revendication 1 est connu du brevet américain US 1, 346, 940.

Un tel ancillaire présente cependant des inconvénients.

Il nécessite en effet de tirer dans la prolongation du lien lui-même qui doit être vrillé pour pouvoir permettre sa mise en tension.

De plus aucun retour manuel de la tension n'est possible avec un tel instrument qui nécessite du coup pour éviter de broyer l'élément osseux un système dynamométrique réglable permettant d'arrêter la mise en tension au delà d'une certaine valeur déterminée.

La présente invention vise à fournir un dispositif de mise en tension d'une bande souple répondant mieux que ceux antérieurement connus aux exigences de la pratique, notamment en ce qu'elle va permettre une plus grande flexibilité, une meilleure sensibilité au

niveau de la mise en tension du fait du concept même des outils utilisés pour une telle mise en tension,

et en ce qu'elle présente des possibilités de réglage très améliorées et ce de façon simple.

Dans ce but l'invention propose notamment un dispositif de mise en tension d'une bande souple pour maintien d'un élément osseux selon la revendication 1.

Les moyens de vissage manuel d'actionnement de la pièce mobile elle-même déportée sur une branche latérale formant un angle, permettent une mise en tension progressive et contrôlée de la bande souple.

Dans des modes de réalisation avantageux, on a de plus recours à l'une et/ou à l'autre des dispositions suivantes :
- les moyens de blocage réglables comportent une crémaillère solidaire de la portion de dispositif, et les moyens de vissage comprennent un système à cliquet avec anti-retour et clé d'actionnement solidaire de la pièce mobile.

La clé d'actionnement autorise une bonne sensibilité de serrage manuel ;
- le système à cliquet comporte un bras de levier de relâche du cliquet. Il est donc très facile de libérer la tension, par exemple pour permettre un nouvel ajustement ;
- les moyens de vissage comportent au moins une vis papillon ;
- le dispositif comporte une pièce de liaison entre tige et portion de dispositif formée par un bloc muni de deux pignons d'axes parallèles entre eux de renvoi d'angle de la dite bande dans son plan, ledit bloc étant solidaire de ladite tige ;
- la pièce de liaison est mobile le long de la tige ;
- l'accrochage de la bande sur la pièce mobile comporte un pignon d'axe parallèle à ceux des pignons du bloc :
- l'angle de ladite portion de dispositif par rapport à la tige est réglable ;
- l'angle α de ladite portion de dispositif par rapport à la tige est compris entre 90° et 130° ;
- l'angle α de ladite portion de dispositif par rapport à la tige est de 90°.

L'invention sera mieux comprise à la lecture de la description qui suit d'un mode de réalisation donné à titre d'exemple non limitatif. La description se réfère aux dessins qui l'accompagnent dans lesquels :
- La figure 1 est une vue en perspective de la bande souple pour maintien d'un élément osseux sur un implant par le biais d'un corps de fixation sur ledit implant.
- La figure 2 est une vue de dessous d'une vertèbre montrant le corps de fixation sur la tige et la bande souple de liaison de la vertèbre avec le corps de fixation.

- La figure 3 est une vue latérale du dispositif selon le mode de réalisation de l'invention plus particulièrement décrit ici.
- La figure 4 est une vue en perspective axionométrique du dispositif de la figure 3.
- La figure 5 est une vue en coupe de la pièce mobile et des moyens de blocage réglable selon le mode de réalisation de l'invention plus particulièrement décrit ici.

Dans la suite de la description on utilisera les mêmes numéros de référence pour désigner les mêmes éléments.

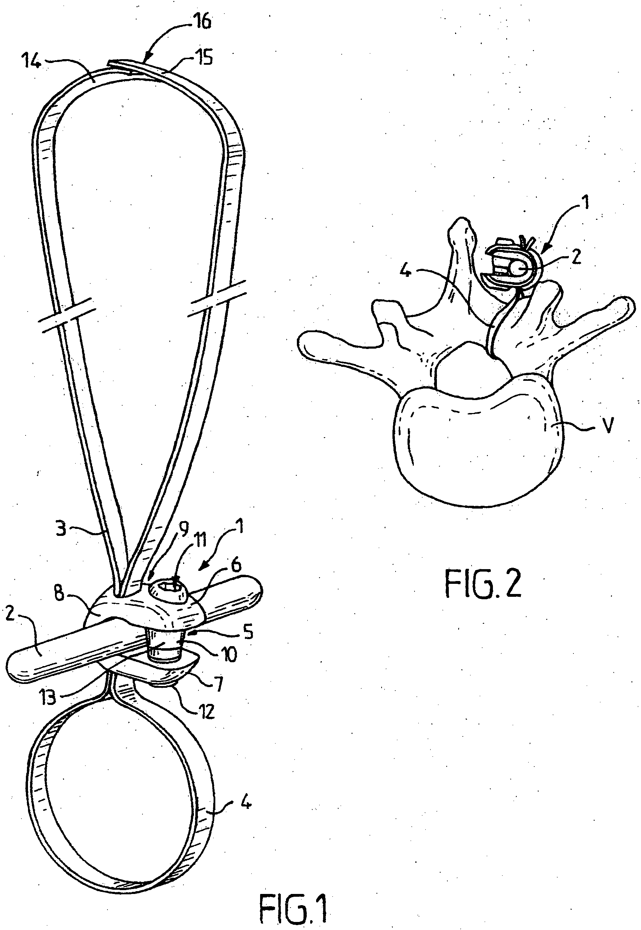

La figure 1 montre un mode de réalisation d'un corps 1 de fixation sur une tige cylindrique 2 et une bande souple 3 en polymère stressé, par exemple en polyester de 6 mm de large et de 30 cm de long pour former la boucle 4.

Des moyens de blocage 5 réglables de la bande souple sur le corps de fixation 3 sont prévus. Le corps de fixation 1 est par exemple formé par une pièce d'un seul tenant formant une pince présentant une section transversale en forme de U, ledit U comprenant deux branches épaisses 6, 7 d'une section transversale sensiblement en forme de demi-ovale, symétrique par rapport à un plan longitudinal est reliées entre elles par une partie de liaison 8 en forme de demi-anneau torique formant d'un côté le fond en demi-cylindre du U et de l'autre côté les parois externes arrondies des branches 6 et 7.

La paroi de fond du U est de forme complémentaire à celle de la tige 2 ou sensiblement complémentaire.

Chaque branche 6, 7 comporte un évidement 9, par exemple en forme de fente large, par exemple cinq à dix fois plus large que l'épaisseur de la tresse pour faciliter son introduction lors de l'opération.

Chaque branche 6 et 7 comporte un orifice cylindrique de passage des moyens 5 de blocage, à savoir un alésage de diamètre D et un orifice cylindrique taraudé de vissage du diamètre d < D.

Les moyens 5 de blocage sont formés par une pièce de liaison 10, ou vis, munie d'un côté d'une tête 11 de passage dans l'alésage du U et de l'autre d'une extrémité 12 de vissage dans ledit orifice cylindrique taraudé en vis à vis.

Dans le mode de réalisation plus particulièrement décrit ici, la tête de la vis 11 comprend une partie supérieure cylindrique et une partie inférieure 13 tronconique vers le bas, agencée pour comprimer la tige 2 au fur et à mesure du vissage de la pièce.

La bande souple 3 est elle jointe à ses autres extrémités 14 et 15 en 16 par exemple par couture, collage ou autres moyens connus en eux-mêmes.

On a représenté sur la figure 2 la vertèbre V telle que fixée par l'intermédiaire de la boucle 4 à la tige 2 via le corps 1 pour permettre la mise en tension de la boucle 4 sur l'os V comme représenté sur la figure 2.

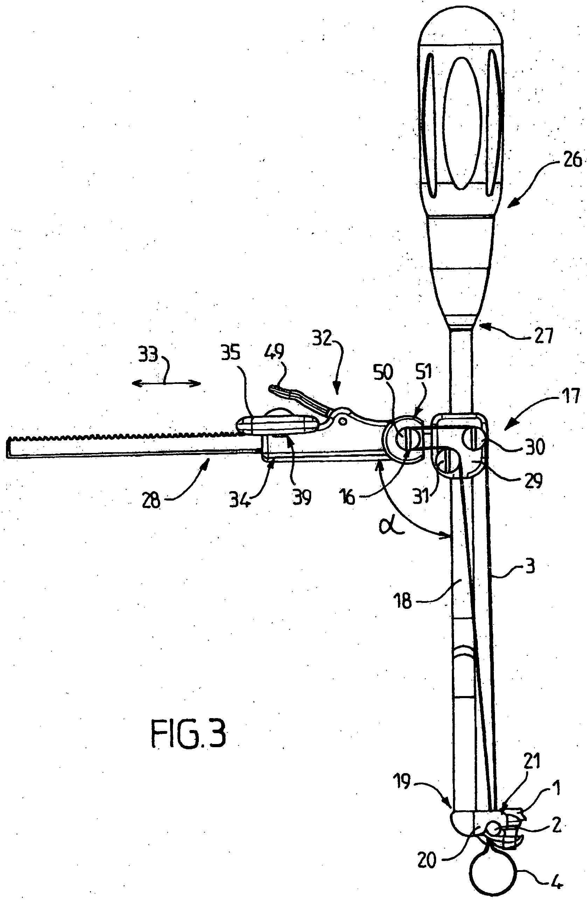

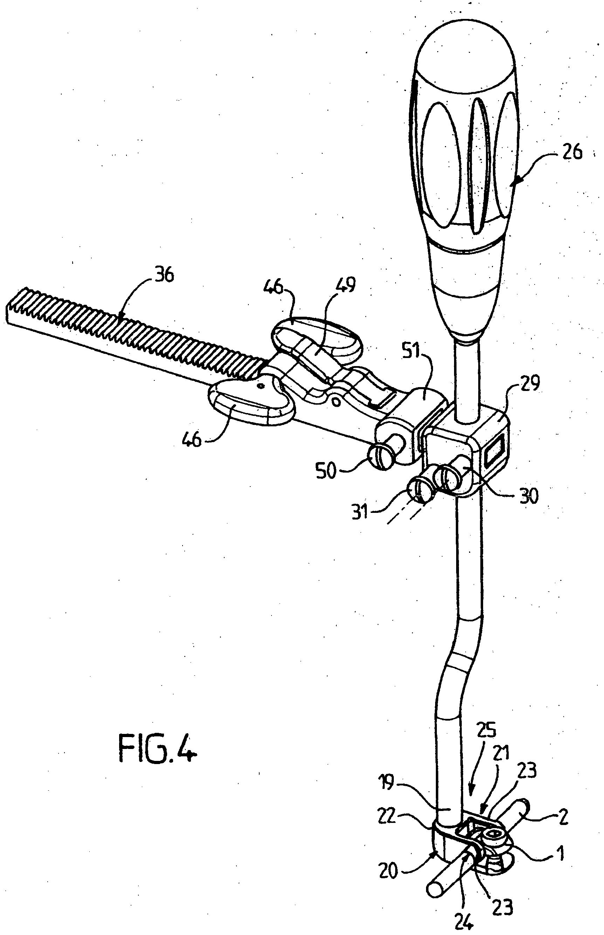

Il va maintenant être décrit le dispositif 17 en référence aux figures 3 et 4.

Le dispositif 17 de mise en tension de la bande souple 3 par l'intermédiaire de la boucle 4, comporte donc une tige 18 longiligne par exemple de 40 cm de long et cylindrique muni à une première extrémité 19 de moyens d'appui 20 par exemple constitués par une pièce solidaire de l'extrémité 19 présentant une partie 21 en crochets cylindriques, de forme complémentaire et agencée pour coopérer avec la barre 2.

Plus précisément la pièce 20 est par exemple formée par une partie de talon 22 solidaire de l'extrémité 19 prolongée de chaque côté par deux branches symétriques parallèles 23 munies à leur extrémité de deux parties d'épaulement 24 agencées pour coopérer avec la tige 2.

Les deux branches 23 sont elles-mêmes séparées par une ouverture 25 dans laquelle vient s'insérer le corps 1 de fixation sur la barre de façon connue en elle-même.

Le dispositif comporte un manche 26 de préhension manuelle dans le prolongement de la tige 18 à sa deuxième extrémité 27.

Le dispositif selon le mode de réalisation de l'invention plus particulièrement décrit ici comporte également une portion 28 formant un angle α avec la tige 18 à laquelle elle est fixée rigidement à un niveau intermédiaire par l'intermédiaire d'une pièce de liaison 29 entre tige et dite portion, formée par un bloc par exemple sensiblement parallélépipédique muni de deux pignons 30 et 31 d'axe parallèle entre eux, perpendiculaires à l'axe de la tige 18, de renvoi d'angle de la bande 3 dans son plan, vers la portion de tige 28.

Il est prévu une pièce 32 mobile en translation (flèche 33) par rapport à la portion 28.

Cette pièce mobile 32 comprend des moyens 34 de blocage réglables de la pièce mobile dans sa dite translation, munis de moyens 35 de vissage.

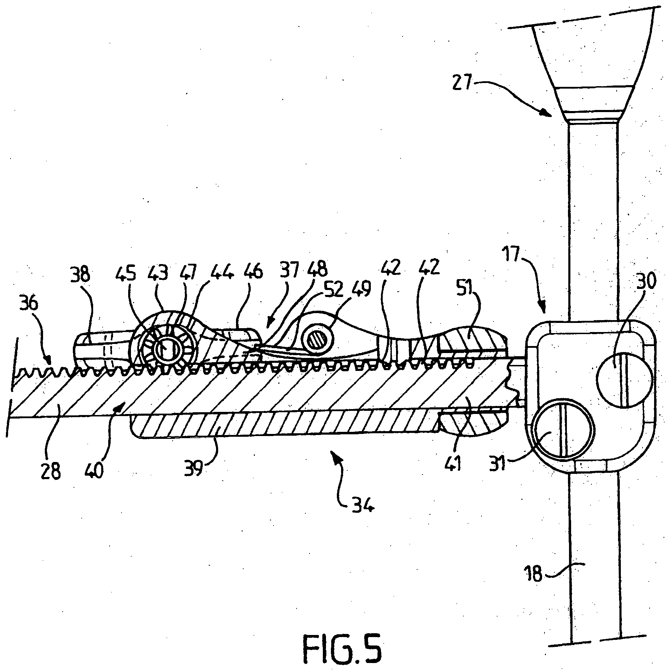

En référence également à la figure 5 les moyens 34 de blocage réglable comportent une crémaillère 36 solidaire de la portion 28, les moyens de vissage 35 comprenant quant à eux un système 37 à cliquet avec anti-retour et clé d'actionnement 38 solidaire de la pièce mobile.

Plus précisément les moyens de blocage réglables comportent un corps 39 percé d'un alésage 40 au travers du quel passe l'extrémité 41 de là portion 28 qui est fixée à la tige 18 par la pièce de liaison 29.

L'extrémité 41 qui comporte sur sa partie supérieure les dents 42 de la crémaillère va coopérer à frottement doux avec l'alésage 40 de la pièce 39 qui comporte dans sa partie supérieure un ergot 43 percé d'un alésage latéral 44 au travers duquel passe l'axe 45 des papillons 46 d'actionnement manuel d'une roue dentée 47 agencée pour coopérer avec les dents 42 de la crémaillère 36 de sorte que lorsque l'on va tourner les vis papillons la pièce mobile 39 va se déplacer transversalement le long de tige.

Des moyens connus en eux-mêmes à cliquet 48 sont prévus actionnables et déverrouillable lorsqu'on lève le levier 49 anti-retour de façon connue en elle-même.

Ceci permet en effet de relâcher le cliquet et de libérer la tension de la bande souple comme cela va maintenant être décrit plus avant.

En effet la bande souple plus exactement les extrémités jointives de cette bande souple en 16 sont solidaires d'un troisième pignon 50 fixé à l'extrémité 51 de la pièce mobile, de sorte que lorsqu'on déplace la pièce mobile vers l'extérieur de la tige, la bande 3 et la boucle 4 se retrouvent mises en tension.

Dans un mode de réalisation avantageux l'angle α entre la portion et la tige est réglable pour être bloqué de façon connue en elle-même.

On va maintenant décrire en référence aux figures 2 et 3 le fonctionnement d'un dispositif selon l'invention.

L'utilisateur va tout d'abord former la boucle 4 autour de l'élément osseux puis glisser les extrémités de cette boucle au travers du corps 1 qui va ensuite être fixé sur la tige en refermant le U de la pièce 1 par l'intermédiaire des moyens de fixation 5 de façon à ce que le glissement des branches de la bande souple puisse encore être réalisé au travers des orifices du corps 8.

La bande souple est ensuite jointe en 16 à ses extrémités par un système de fermeture connu en lui-même et est ensuite passée dans les pignons de renvoi ou cabestans 30, 31 et 50.

Le dimensionnement de la bande souple aura été au préalable calculé de façon à ce que la bande soit à peu près en position tendue. Au cas où celle-ci serait plus importante, il est possible aussi de bouger par exemple dans un mode de réalisation la pièce de fixation de la portion 28 sur ladite tige de façon à obtenir la tension initiale recherchée.

La tension s'effectue ensuite en tournant les papillons 46, ce qui entraîne la roue dentée 47 qui engraine sur la crémaillère.

L'anti-retour est assuré par le levier qui s'appuie à l'aide d'un ressort 52 sur les dents de la même crémaillère.

La tension peut être relâchée en agissant sur le levier 49 afin de permettre un retour de la pièce mobile ou chariot.

Comme il va de soi et comme il résulte également de ce qui précède, la présente invention n'est pas limitée aux modes de réalisation plus particulièrement décrits. Elle en embrasse au contraire toutes les variantes qui tombent sous la portée des revendications et notamment celles ou il n'y a qu'une seule clé papillon, celle où les moyens de vissage sont différents et/ou celles où les pièces intermédiaires 29, 51 sont constituées différemment.

## Revendications

1. Dispositif (17) de mise en tension d'une bande souple (3) sur un élément osseux comprenant une extrémité d'appui agencée pour être située du côté de l'élément osseux, une pièce de liaison (29) de renvoi d'angle de la bande et reliant l'extrémité d'appui avec une portion (28) du dispositif propre à former un angle avec l'extrémité d'appui, une pièce mobile d'accrochage de la bande souple sur ladite portion (28), et des moyens de blocage réglable de la pièce mobile en translation par rapport à ladite portion de dispositif comprenant des moyens (35) de vissage manuel propre à actionner la pièce mobile, **caractérisé en ce que**
le dispositif comprend une tige munie de ladite extrémité d'appui, ladite extrémité d'appui étant munie de moyens d'appui (20) sur un implant pour maintien de l'élément osseux,
**en ce qu'**il comprend un manche de préhension manuel et **en ce que** la pièce de liaison (29) de renvoi d'angle de la bande est fixée rigidement à la portion (28), à un niveau intermédiaire de la tige.

2. Dispositif selon la revendication 1, **caractérisé en ce que** les moyens (34) de blocage réglables comportent une crémaillère (36) solidaire de la portion (28) de dispositif, et **en ce que** les moyens (35) de vissage comprennent un système (37) à cliquet avec anti-retour et clé d'actionnement (38) solidaire de la pièce (32) mobile.

3. Dispositif selon la revendication 2, **caractérisé en ce que** le système (37) à cliquet comporte un bras de levier (19) de relâche du cliquet.

4. Dispositif selon l'une quelconque des revendications précédentes, **caractérisé en ce que** les moyens (35) de vissage comportent au moins une vis papillon (46).

5. Dispositif selon l'une quelconque des revendications précédentes, **caractérisé en ce que** la pièce (29) de liaison est entre tige et portion de dispositif et est formée par un bloc muni de deux pignons (30, 31) d'axes parallèles entre eux de renvoi d'angle de la dite bande dans son plan, ledit bloc étant solidaire de ladite tige.

6. Dispositif selon la revendication 5, **caractérisé en ce que** la pièce (29) de liaison est mobile le long de la tige.

7. Dispositif selon l'une quelconque des revendications 5 et 6, **caractérisé en ce que** l'accrochage de la bande sur la pièce mobile comporte un pignon (50) d'axe parallèle a ceux des pignons du bloc.

8. Dispositif selon l'une quelconque des revendications précédentes, **caractérisé en ce que** l'angle de ladite portion de dispositif par rapport à la tige est réglable.

9. Dispositif selon l'une quelconque des revendications précédentes, **caractérisé en ce que** l'angle α de ladite portion de dispositif par rapport à la tige est compris entre 90° et 130°.

10. Dispositif selon la revendication 9, **caractérisé en ce que** l'angle a de ladite portion de dispositif par rapport à la tige est de 90°.

## Patentansprüche

1. Vorrichtung (17) zum Spannen eines flexiblen Bandes (3) auf einem Knochenelement mit einem Stützende, das so angeordnet ist, dass es sich auf der Seite des Knochenelements befindet, einem Verbindungsstück (29) einer Eckumlenkung des Bandes, das das Stützende mit einem Abschnitt (28) der Vorrichtung verbindet, welcher geeignet ist, mit dem Stützende einen Winkel zu bilden, einem beweglichen Teil zum Einhängen des flexiblen Bandes an dem Abschnitt (28) und einstellbaren Mitteln für die Arretierung des verschieblichen Teils in Bezug auf den Vorrichtungsabschnitt mit Mitteln (35) für eine Handverschraubung, die geeignet ist, das bewegliche Teil zu betätigen,
**dadurch gekennzeichnet,**
**dass** die Vorrichtung einen mit dem Stützende versehenen Stift aufweist, wobei das Stützende mit Mitteln (20) zum Abstützen auf einem Implantat zum Halten des Knochenelements versehen ist, dass sie einen mit der Hand ergreifbaren Stiel aufweist und dass das Verbindungsstück (29) einer Eckumlenkung des Bandes mit dem Abschnitt (28) auf einer Zwischenhöhe des Stifts starr verbunden ist.

2. Vorrichtung nach Anspruch 1,
**dadurch gekennzeichnet,**
**dass** die einstellbaren Arretierungsmittel (34) eine mit dem Vorrichtungsabschnitt (28) fest verbundene Zahnstange (36) aufweisen und die Verschraubungsmittel (35) ein System (37) mit einer Sperrklinke und einem mit dem beweglichen Teil (32) fest verbundenen Betätigungsschlüssel (38) aufweisen.

3. Vorrichtung nach Anspruch 2,
**dadurch gekennzeichnet,**
**dass** das Klinkensystem (37) einen Hebelarm (19) zum Entspannen der Klinke aufweist.

4. Vorrichtung nach einem der vorhergehenden Ansprüche,
**dadurch gekennzeichnet,**
**dass** die Verschraubungsmittel (35) mindestens eine Flügelschraube (46) aufweisen.

5. Vorrichtung nach einem der vorhergehenden Ansprüche,
**dadurch gekennzeichnet,**
**dass** das Verbindungsstück (29) zwischen Stift und Vorrichtungsabschnitt angeordnet ist und durch einen Block gebildet ist, der mit zwei parallel zueinander verlaufenden Achsritzeln (30, 31) einer Eckumlenkung des Bandes in dessen Ebene versehen ist, wobei der Block mit dem Stift fest verbunden ist.

6. Vorrichtung nach Anspruch 5,
**dadurch gekennzeichnet,**
**dass** das Verbindungsstück (29) entlang des Stifts beweglich ist.

7. Vorrichtung nach einem der Ansprüche 5 und 6,
**dadurch gekennzeichnet,**
**dass** die Einrichtung zum Einhängen des Bandes an dem beweglichen Teil ein Achsritzel (50) aufweist, das parallel zu denjenigen der Ritzel des Blocks verläuft.

8. Vorrichtung nach einem der vorhergehenden Ansprüche,
**dadurch gekennzeichnet,**
**dass** der Winkel zwischen dem Vorrichtungsabschnitt und dem Stift einstellbar ist.

9. Vorrichtung nach einem der vorhergehenden Ansprüche,
**dadurch gekennzeichnet,**
**dass** der Winkel α zwischen dem Vorrichtungsabschnitt und dem Stift zwischen 90° und 130° liegt.

10. Vorrichtung nach Anspruch 9,
**dadurch gekennzeichnet,**
**dass** der Winkel α zwischen dem Vorrichtungsabschnitt und dem Stift 90° beträgt.

## Claims

1. Device (17) for tensioning a flexible band (3) on a bone element comprising a bearing end arranged so as to be situated on the side of the bone element, a connecting part (29) changing the angle of the band and connecting the bearing end with a portion (28) of the device capable of forming an angle with the bearing end, a mobile part for attaching the flexible band on said portion (28), and means for adjustable locking of the part which is mobile in translation in relation to said portion of the device comprising manual screwing means (35) capable of actuating the mobile part,
**characterised in that**
the device comprises a rod provided with said bearing end, said bearing end being provided with bearing means (20) for bearing on an implant to hold the bone element,
**in that** it comprises a handle for manual gripping and **in that** the connecting part (29) changing the angle of the band is fixed rigidly to the portion (28) at an intermediate level of the rod.

2. Device according to claim 1, **characterised in that** the means (34) for adjustable locking comprise a rack (36) which is integrally locked to the portion (28) of the device, and **in that** the screwing means (35) comprise a system (37) with a ratchet with a non-return means and an actuating key (38) integrally locked to the mobile part (32).

3. Device according to claim 2, **characterised in that** the system (37) with a ratchet comprises a lever arm (19) for releasing the ratchet.

4. Device according to any one of the preceding claims, **characterised in that** the screwing means (35) comprise at least one butterfly screw (46).

5. Device according to any one of the preceding claims, **characterised in that** the connecting part (29) is between the rod and the portion of the device and is formed by a block provided with two pinions (30, 31) with mutually parallel axes changing the angle of said band in its plane, said block being integrally locked to said rod.

6. Device according to claim 5, **characterised in that** the connecting part (29) is mobile along the rod.

7. Device according to any one of claims 5 and 6, **characterised in that** the attachment of the band on the mobile part comprises a pinion (50) with its axis parallel with the axes of the pinions of the block.

8. Device according to any one of the preceding claims, **characterised in that** the angle of said portion of the device in relation to the rod is adjustable.

9. Device according to any one of the preceding claims, **characterised in that** the angle α of said portion of the device in relation to the rod lies between 90° and 130°.

10. Device according to claim 9, **characterised in that** the angle α of said portion of the device in relation to the rod is 90°.
